(19) Europäisches Patentamt
European Patent Office
Office européen des brevets

(11) **EP 4 256 324 B1**

(12) **EUROPEAN PATENT SPECIFICATION**

(45) Date of publication and mention
of the grant of the patent:
**17.12.2025 Bulletin 2025/51**

(21) Application number: **21839280.1**

(22) Date of filing: **02.12.2021**

(51) International Patent Classification (IPC):
**G01N 33/14** $^{(2006.01)}$     **G01N 33/00** $^{(2006.01)}$
**G01N 1/22** $^{(2006.01)}$

(52) Cooperative Patent Classification (CPC):
**G01N 33/146; G01N 33/0006;** G01N 33/0018;
G01N 2001/2229

(86) International application number:
**PCT/IT2021/000055**

(87) International publication number:
**WO 2022/118346 (09.06.2022 Gazette 2022/23)**

(54) **METHOD AND APPARATUS FOR MEASURING THE ALCHOHOLIC CONTENT OF AQUEOUS SOLUTIONS BY MEANS OF GAS SENSORS**

VERFAHREN UND VORRICHTUNG ZUR MESSUNG DES ALCHOHOLISCHEN GEHALTES WÄSSRIGER LÖSUNGEN MITTELS GASSENSOREN

PROCÉDÉ ET APPAREIL DE MESURE DE TENEUR ALCOOLIQUE DE SOLUTIONS AQUEUSES AU MOYEN DE CAPTEURS DE GAZ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.12.2020 IT 202000029444**

(43) Date of publication of application:
**11.10.2023 Bulletin 2023/41**

(73) Proprietor: **ENHANCED SYSTEMS & TECHNOLOGIES S.R.L.**
**83100 Avellino (IT)**

(72) Inventors:
• **CACCAVO, Diego**
**83100 Avellino (AV) (IT)**
• **LAMBERTI, Gaetano**
**83100 Avellino (AV) (IT)**

(74) Representative: **Masetti Zannini de Concina, Alessandro**
**Akran Intellectual Property s.r.l.**
**Piazza del Gesù, 46**
**00186 Roma (IT)**

(56) References cited:
WO-A1-2019/211813     WO-A1-90/13026
IT-A1- 201700 138 957

• **ANGHEL M A ET AL: "The influence of CO 2 in the measurements of mass concentration of alcohol in exhaled breath using dual IR & EC technology", ELECTRICAL AND POWER ENGINEERING (EPE), 2012 INTERNATIONAL CONFERENCE AND EXPOSITION ON, IEEE, 25 October 2012 (2012-10-25), pages 805 - 809, XP032425165, ISBN: 978-1-4673-1173-1, DOI: 10.1109/ICEPE.2012.6463582**
• **DUBOWSKI KURT M.: "Breath-Alcohol Simulators: Scientific Basis and Actual Performance", JOURNAL OF ANALYTICAL TOXICOLOGY, vol. 3, no. 5, 1 September 1979 (1979-09-01), US, pages 177 - 182, XP055826949, ISSN: 0146-4760, DOI: 10.1093/jat/3.5.177**
• **MANDENIUS CARL FREDRIK ET AL: "AN ONLINE SENSOR FOR MONITORING OF ETHANOL IN BEER", JOURNAL OF THE INSTITUTE OF BREWING., vol. 90, no. 2, 4 March 1984 (1984-03-04), GB, pages 77 - 80, XP055896664, ISSN: 0046-9750, DOI: 10.1002/ j.2050-0416.1984.tb04241.x**

**Description**

**Field of the invention**

The present invention relates to a method and an apparatus for determining the alcohol content of aqueous solutions by means of gas sensors. More specifically, the invention relates to a method and a device for determining the alcohol content of aqueous solutions by analyzing the vapor phase that overhangs the solutions themselves by means of gas sensors, which allow accurate and reliable measurements to be made, with relatively short analysis times. The method and the apparatus are particularly suitable for the quantification of ethyl alcohol in binary water-ethanol solutions, as well as in commercial alcoholic beverages.

**Background of the invention**

The determination of the concentration of ethyl alcohol in aqueous solutions is of fundamental importance for the production and marketing of alcoholic beverages. The strong industrial interest in the quantification of alcohol content is evident from the large number of methods and devices that have been developed over the years for these measurements. The main methods by which the alcohol content of beverages is measured are listed below:

- method by distillation (official method), followed by pycnometry, electronic densimetry or with hydrostatic balance;
- ebulliometric method;
- methods for chemical oxidation;
- method for enzymatic oxidation;
- advanced analytical techniques - fluorescence spectroscopy, refraction index, transmission spectrum, and chromatography (HPLC);
- methods based on the analysis of the vapor phase using gas sensors.

An official version of the distillation method was published in the Official Journal of the European Communities (Commission Regulation (EC) No. 2870/2000 of 19 December 2000, which defines the reference Community methods of analysis applicable in the spirit drinks sector. self). This method involves a first purification phase by distillation of the solution to be analyzed (evaporation and almost complete condensation of the starting solution), thus removing all the other components potentially interfering with subsequent analyzes in the volatiles or in the bottom residue. Once the distillate has been obtained, it will be brought back to the volume of the starting solution, thus reestablishing the initial alcohol-water ratio, and then subjected to analysis for the calculation of the alcohol content. The second phase, which is the actual measurement of the alcohol content, can be carried out by means of pycnometry, electronic densimetry or densimetry with hydrostatic balance. All three of these methods provide, following the evaluation of the density, the use of tables for the conversion of this value into alcohol.

The distillation method described above has major practical drawbacks mainly linked to a considerable consumption of resources, both in terms of equipment to be equipped with and to be able to use, and in terms of time required to obtain the results.

The ebulliometric method (Zoecklein, BW, Production Wine Analysis, Springer-Verlag, New York Inc., US, 2012) is based on the use of a ebulliometer, an approximate instrument for measuring the alcohol content that exploits the difference between the boiling temperatures of ethanol and water. Depending on the value of the temperature at which boiling is recorded, the alcohol content of the solution is determined: the lower the boiling temperature, the greater the alcohol content of the mixture. The ebulliometer generally provides well reproducible data for pure water-ethanol solutions, but the presence of other components (for example sucrose > 5g/L) produces significant errors in the measurement.

The method based on chemical oxidation exploits the oxidation of ethanol to acetic acid by reaction with potassium dichromate and sulfuric acid, in order to determine by colorimetric the excess of unreacted dichromate in solution (Williams, MB and Reese, HD, Colorimetric determination of ethyl alcohol, Anal. Chem. 1950, 22 (12): 1556-1561). The chromophore element is s-diphenyl carbazide which, by binding to the dichromate ions, forms a complex with a strong purple color. Therefore, once the initial and final quantities of di-chromate are known and the stoichiometry of the oxidation reaction known, it is possible to evaluate the quantity of alcohol reacted in solution, and from this the alcoholic degree.

The method described above suffers from strong limitations, mainly due to the preparation of the reagents, which must take place according to different recipes depending on the alcohol concentration to be determined, thus requiring an estimate of the alcohol content before the measurement.

The methods for enzymatic oxidation are based on the use of an enzyme, alcohol oxidase, capable of reacting even with very small quantities of ethanol, in the presence of oxygen, to produce hydrogen peroxide and acetaldehyde. Such a method is described, for example, in the European patent EP 0357027 B1 in the name of New Oji Paper Co. (1990), entitled "Enzyme electrode and method for determination of alcohol content using the same". Once the enzymatic reaction has taken place, once the reagents fed are known, it is possible to measure the hydrogen peroxide or acetaldehyde produced by the reaction, or the oxygen consumed by said reaction.

The greatest limitation of the methods of determining the alcohol content by enzymatic method consists in the fact that the alcohol concentration in the samples must always be low enough for the alcohol to be oxidized by enzymes.

Advanced analysis techniques include all those high-tech methods that require specific expertise on the part of the operators. Among these, for example, fluorescence spectroscopy is based on the detection of the fluorescence intensity in a sample containing alcohol to which a fluorescent agent is mixed. In US patent application US 2004/0157334 (Barashkov et al), the intensity of the fluorescence is determined in a specific spectral region, and compared with those of samples of known alcohol strength, to determine the concentration of the analyzed sample.

Another advanced technique used for the determination of ethanol, usually in addition to other compounds (such as carboxylic acids, sugar, glycerol) is HPLC (high performance liquid chromatography). This is based on the separation of different components in solution depending on their ability to be adsorbed on a substrate, in an adsorption column. Once separated, the different substances are detected and their concentration quantified thanks to the deter-mination of absorbance in the UV spectrum (Falqué López, E. and Fernández Gómez, E., "Simultaneous determination of the major organic acids, sugars, glycerol, and ethanol by HPLC in grape musts and white wines ", J. Chromatogr. Sci. 1996, 34 (5): 254-257).

The evaluation of transmittance and refractive index can be another method for determining the concentration of alcohol in solution. This technique is based on the principle according to which, when light propagates in a solution, the transmission and reflection properties are different, depending on the concentration of the components in the solution (Zhang, Y., et al., "A new method study of spectral measurement and prediction based on the non-linear solution concentration of alcohol", Physica B: Condensed Matter 2017, 516: 32-35).

The above methods, which share the fact that they require complex and expensive equipment as well as highly qualified personnel, are difficult to use in daily industrial practice and during production processes.

Procedures based on the analysis of the vapor phase using gas sensors have already been proposed for the determination of the concentration of alcohol in aqueous solutions. The known methods are based on the use of sensors for immersion gases, or on the analysis of gas streams after a step of blowing into the solution to be analyzed. In the use of sensors for immersion gases, semipermeable membranes are used, for example made of silicone, which are able to let the ethanol permeate from the solution towards a semiconductor sensor placed downstream of the membrane (Vorlop, KD, et al ., "On-line measurement of ethanol with a gas-sensor-dip-electrode", Biotechnology Letters 1983, 5 (8): 509-514). This technique, which also saw the development of a commercial product (Vernier. "Ethanol Sensor Eth-Bta." Retrieved 09/15/2017, from https://www.vernier.com/products/sensors/eth- bta/), has major limitations linked to the concentration range of analyzable ethanol, which cannot exceed 3% vol. due to sensor saturation.

Methods and devices that provide for the analysis of post-blowing gaseous streams in the solution to be analyzed exploit the fact that the gaseous stream (e.g. air) bubbled in the liquid is enriched with alcohol (and water). The pre-treated gas is analyzed using gas sensors, often after passing through semipermeable membranes to remove excess moisture. An example is the device described in US patent 5091155 (Takayama et al.), Which is immersed in a liquid (a solution of water and alcohol), leaving the upper part (closed on the surface by a cap) in air. A tube is passed through the cap, so that it is partially immersed in the liquid and, by means of a device for pumping air, the latter is pushed first into the tube and then into the solution to be analyzed. The air flow and the height at which the tube is immersed must be such that the bubbles formed are able to absorb a sufficient amount of alcohol during their ascent. After absorbing the alcohol, the gas passes, rising, through a temperature sensor for gas placed inside the cap, in the vicinity of a device for the oxidation of the alcohol evaporated from the liquid. This sensor therefore records the temperature variation associated with the oxidation of alcohol which, after an appropriate calibration, can be related to the alcohol concentration within the sample solution.

A device similar to that described by Takayama can also be equipped with a semiconductor alcohol sensor, or a platinum catalytic sensor which, after suitable calibration, is capable of measuring the alcohol concentration in the starting solution. These devices are often equipped with auxiliary sensors, in particular temperature and humidity sensors, which are used to correct the measurement. For example, the international patent application publ. n. WO 90/13026 in the name of Crisp In-instrumentation Ltd. and the publication of the same research group (Browne, A. et al., "Development of a Sturdy, Portable Instrument for the Rapid Deter-mination of Alcohol in Beer", ASBC Journal 1991, 49 (2): 74-77) describe a method and a device for measuring the concentration of ethanol in beverages, in which the method consists of the following steps: a) preparing hydroalcoholic solutions at known alcohol concentrations, for calibration; b) whirl air through each solution to remove at least part of the alcohol present in the solution; c) analyzing the concentration of ethanol in the gas phase using gas sensors; d) construct a calibration curve; e) analyzing samples with unknown ethanol concentrations. The device in question was specifically designed for determining the alcohol content of beers, and therefore is able to determine alcohol in a concentration range between 0.5 and 10.0% v/v.

The article by Ab Mutalib and colleagues (Ab Mutalib, NA et al., IIUM-Fabricated Portable Electronic Nose for Halal Authentication in Beverages, 5th International Conference on Information and Communication Technology for the Muslim World (ICT4M), 2013, 1-4 ) describes a device for the detection and quantification of ethyl alcohol in solutions with a low alcohol content, usable for the halal certification of beverages. The device carries out the aspiration of the head space above the drink and its dilution with air through a fan. As reported in the same work, however, this device was developed to determine the possible presence of alcohol in beverages in order to exclude them from those admitted for consumption for the Islamic world, and therefore has a sensitivity that allows for the appreciation of ethanol concentrations even of 0.1% vol., But does not consider hydroalcoholic solutions with an alcohol content above 10% vol.

A method and a device similar to the previous ones have been described in the Italian patent application n. 102017000138957 in the name of Caccavo et al. This device analyzes the vapor phase which overhangs the hydroalcoholic solution whose alcoholic degree is to be determined after having mixed it with air. By means of calibration with solutions of known title it is possible to trace the concentration of hydroalcoholic solutions of unknown title. Specifically, the method described in the patent application is divided into the following steps: a) preparation of hydroalcoholic solutions with a known concentration of ethanol; b) aspiration and dilution of the vapor phase overlying the aforementioned hydroalcoholic solutions; c) analysis of the gas phase concentration of ethanol using gas sensors; d) construction of the calibration curve that links the read concentration to the known one in the liquid phase; e) analysis, preferably under the same operating conditions (temperature, humidity, volumetric flow rates and dilution ratios) used for the construction of the calibration curve, of samples with unknown concentration.

The methods based on the analysis of the concentration of ethanol in the vapor phase in the headspace above the solution to be analyzed have several disadvantages, ranging from the necessary pre-treatment of the sample (e.g. degassing and addition of anti-foaming agents, for example in the case of beers), due to the presence of high and highly variable humidity in the stream to be analyzed, with repercussions on the sensor response and the need for a dehumidification phase.

Furthermore, as already noted, many of these methods have the big limit linked to the maximum measurable alcoholic degree which, in order to remain within the measurement limit of the sensors used, turns out to be extremely low.

Furthermore, the known methods and devices have two major drawbacks which have precluded their industrial exploitation:

1) the presence of volatile organic compounds, in particular in commercial beverages (esters, organic acids, aldehydes, carbonyl compounds, terpenes, etc.) makes the calibration of the instrument inaccurate with pure hydroalcoholic solutions, leading to the overestimation of the alcohol content;

2) the concentration of the gaseous phase above the solutions is strongly dependent on the temperature.

The only work mentioned above to have taken into account the two problems is the work of Browne, Buckee et al., already cited, in which the problem of volatile organic compounds was circumvented by calibrating the device with products similar to those to be analyzed, in particular beers of different alcoholic strength for the analysis of beers with unknown alcoholic content. This, of course, reduces the potential of the method and the tool, binding them to use for a specific drink. Furthermore, to take into account the effect of temperature, the calibration (with a specific beverage) must be carried out at multiple temperatures, obtaining a calibration surface in which the sensor signal is connected to both the alcohol concentration of the solution and the temperature.

Considering all the methods for the determination of the alcoholic strength described so far, it is noted that the techniques for distillation, ebulliometric, for chemical and enzymatic oxidation, as well as those that exploit advanced analysis techniques, require laboratories specialized in these methods, long times of analysis, sample pretreatment and highly specialized personnel, which makes these methods difficult to apply to industrial practice. On the other hand, the techniques based on the analysis of the vapor phase using gas sensors, if properly equipped with instrumentation capable of transducing the sensor signal, can be a valid method for the analysis of alcoholic solutions even at an industrial level. However, the methods and devices proposed so far have several disadvantages that have limited their industrial use, including the need for pre-treatment of the samples, the need for dehumidification of the gas stream and above all the impossibility of correctly measuring alcoholic solutions with concentrations higher than ten percentage points. Furthermore, the presence of volatile organic compounds in commercial alcoholic beverages, as well as the temperature variation of the samples, are still unsolved problems.

In light of the aforementioned prior art, the present invention therefore aims to develop a method and a device capable of analyzing in a short time, through a simple procedure that does not require specific knowledge and complex devices, the vapor phase that overcomes the alcoholic solutions through the use of gas sensors, in such a way as to overcome the problems of the previous methods, and reducing the interference of volatile organic compounds and the impact of temperature variations.

**Summary of the invention**

In order to overcome the limitations of the prior art, according to a first aspect of the invention, a method has been developed capable of providing a rapid and precise evaluation of the ethanol content of hydroalcoholic solutions, including commercial beverages, by measuring the concentration of alcohol in a corresponding vapor phase.

According to another aspect of the invention, the proposed method has been re-produced in practice thanks to a specially designed device, suitable to be used for the determination of the alcohol content of solutions and beverages of any strength.

In the method of the invention, the solution to be analyzed is first diluted with water in the liquid phase, so as to minimize the partial pressure of the volatile organic compounds in the corresponding vapor phase. The resulting vapor phase can then be sucked in by means of a device, such as a vacuum pump, and sent to a device capable of mixing this stream with air, such as a Venturi injector. The mixture of air enriched in alcohol can thus be sent to gas sensors for ethanol analysis, such as semiconductor sensors, to then be discharged into the environment.

The dilution of the vapor phase with air has the advantages of bringing the alcohol content back into the measurement range of commercially available gas sensors and limiting the influence of the humidity of the stream to be analyzed on the sensor response. By alternating loading phases, in which the air stream is mixed with alcohol vapors, and cleaning phases, in which the air stream is sent pure, and possibly heated, to the sensors, it is possible to alternate measurement stages with regeneration stages. sensors, if needed.

Through the use of a calculation module equipped with analog and/or digital pins for outgoing and incoming communications, such as a single board microcontroller and appropriate circuitry, it is possible to modulate the inlet air flow and the mixing, and analyze the response of the sensors so as to be able to quantify the alcohol concentration present in the gas phase. The sensor response can be correlated to the alcohol concentration in the gas phase through calibration curves constructed from the analysis of solutions whose liquid phase has a known alcoholic concentration and whose concentration of ethanol in the vapor phase has been obtained by means of thermodynamic models and/or by means of known semi-empirical models.

Therefore, for the analysis of a sample of unknown title, the sensor response is linked to the concentration in the gas phase, and then, by means of thermodynamic and/or semi-empirical models already mentioned, it is possible to calculate the unknown ethanol concentration in the liquid phase. In this way the effect of volatile organic compounds is canceled, the effect of temperature is taken into account (through the pure thermodynamic calculation or through the use of semi-empirical equations) and avoids having to dehumidify the stream sent to the sensors.

### Brief description of the drawings

The specific features of the invention, as well as its advantages, will be more evident with reference to the attached figures, in which:

**Figure 1** shows a process diagram (*PFD: Process Flow Diagram*) of the device according to the invention in the preferred measurement configuration;
**Figure 2** shows a flow diagram (*P&ID: Piping and Instrumentation Diagram*) of the device of Figure 1 in the preferred measurement configuration;
**Figure 3** shows the sensor response of the device of Figure 2 following measurement cycles of three known titer solutions used for calibration: 2, 10 and 20% vol. diluted 1 to 25 in water;
**Figure 4** shows the power law relationship that can be obtained between the maximum voltage recorded by the sensors for solutions with a known strength and the vapor phase concentration of ethanol, which can be calculated when the alcoholic strength of the solutions is known and their temperature.

### Detailed description of the invention

Therefore, the present invention specifically provides a method for determining the alcoholic degree of hydroalcoholic solutions or alcoholic beverages, comprising the following operations:

A) Calibration:

a) preparation of hydroalcoholic solutions with a known concentration of ethanol, said concentration of ethanol being in the range from 0 to 100% vol. of ethanol, and having a known temperature;
b) calculation of the concentration in the vapor phase of ethanol using liquid-vapor equilibrium curves for non-ideal mixtures, or using semi-empirical equations, such as Dubowski's formula;
c) aspiration and dilution in a gaseous phase of the vapor phase which overhangs the aforesaid hydroalcoholic solutions;
d) analysis of the gas phase concentration of ethanol using gas sensors;
e) construction of the calibration curve that links the sensor response to the concentration in the gaseous phase;

B) Analysis of samples of unknown alcohol content: unknown samples:

f) dilution of the sample in water (e.g. 1:50) to reduce the partial pressure of volatile organic compounds in the vapor phase, and measurement of its temperature;

g) aspiration and dilution in a gaseous phase of the vapor phase that overhangs the hydroalcoholic solution resulting from operation f);

h) analysis of the concentration of ethanol in the gaseous phase resulting from operation g) using gas sensors and on the basis of the calibration curve obtained from operation e);

i) calculation of the concentration of ethanol in the liquid phase using the same liquid-vapor equilibrium curves for non-ideal mixtures or semi-empirical equations used for operation b).

If, for example, one wishes to determine the concentration of ethanol in the vapor phase for water-ethanol solutions at a known concentration with the semi-empirical method that uses Dubowski's formula, the following equation must be applied:

$$C_{ethanol}^{VAP} = 4.145 \times 10^{-2} \times C_{ethanol}^{LIQ} \times \exp(0.06583\,T)$$

where $C_{ethanol}^{VAP}$ is the concentration of ethanol (in mg/dm³) of the vapor phase above the hydroalcoholic solution,

$C_{ethanol}^{LIQ}$ is the concentration of ethanol [g/dm³] in the corresponding liquid solution and T is the temperature in °C.

For a more rigorous description of the liquid-vapor equilibrium, it is necessary to start from the definition of physical equilibrium, considering the equality of the chemical potentials of the i-th species between the two phases. It can be shown that, considering ideal gases and non-ideal liquids, the "modified" Raoult's law holds: $y_i P = x_i P_i^{sat} \gamma_i$, where $y_i$ and $x_i$ are the molar fractions of the i-th species respectively in the vapor phase and in the liquid phase, $P$ is the total pressure of the system, $P_i^{sat}$ is the saturation pressure of the i-th component and $\gamma$ is the activity coefficient, index of the deviation of the system from ideality. The system concerned by the present invention can be approximated to a binary system with inert in the gas phase (air-ethanol-water) and to binary system in the liquid phase (ethanol-water). The mass fractions in the vapor phase and in the liquid phase are subject to constraints $y_e + y_w + y_a = 1$ and $x_e + x_w = 1$, where the subscripts e, w and a respectively indicate ethanol, water and air. Given the composition of the liquid phase, by solving Raoult's law for water and ethanol, using for example the Wilson activity coefficients and considering a system at constant total pressure system (1 atm), it is possible to calculate all the molar fractions ($y_i$) which are in equilibrium with a given liquid composition of the liquid phase ($x_i$) at a given temperature (and pressure). Given the molar fractions, the calculation of the concentration of ethanol in the vapor phase is possible through the law of ideal gases and the molecular weight of the ethanol itself. By way of example, for the binary system in the liquid phase (water, alcohol) and the tertiary system in the vapor phase (water, alcohol, air), given the concentration of the liquid phase $x_e$, the total pressure of the system ($P = 1$ atm) and the temperature, the equations to solve are as follows:

$$\begin{cases} P\,y_e = x_e P_e^{sat} \gamma_e \\ P\,y_w = x_w P_w^{sat} \gamma_w \\ y_e + y_w + y_a = 1 \\ x_e + x_w = 1 \end{cases}$$

The saturation pressures of ethanol and water can be calculated using the Antoine equation:

$$ln\left(P_e^{sat}\,(kPa)\right) = 16.8958 - \frac{3795,17}{T\,(K) - 42.2320}$$

$$ln\left(P_w^{sat}\,(kPa)\right) = 16.3872 - \frac{3885.7}{T\,(K) - 42.9800}$$

The activity coefficients may be calculated using the Wilson equation:

$$ln(\gamma_e) = -ln(x_e + \Lambda_{ew}x_w) + x_w\left(\frac{\Lambda_{ew}}{x_e + \Lambda_{ew}x_w} - \frac{\Lambda_{we}}{x_w + \Lambda_{we}x_e}\right)$$

$$ln(\gamma_w) = -ln(x_w + \Lambda_{we}x_e) - x_e\left(\frac{\Lambda_{ew}}{x_e + \Lambda_{ew}x_w} - \frac{\Lambda_{we}}{x_w + \Lambda_{we}x_e}\right)$$

where $\Lambda_{ew}$ e $\Lambda_{we}$ are two functions of the temperature which take into account the interactions between ethanol and water. $\Lambda_{ew}$ and $\Lambda_{we}$ may be calculated by means of the following semi-empirical equations:

$$ln(\Lambda_{ew}) = -2.5035 + \frac{346.1512}{T(K)}$$

$$ln(\Lambda_{we}) = -0.0503 - \frac{69.6372}{T(K)}$$

Therefore, for each $x_e$ it is possible, if the temperature is known as well, to calculate the activity coefficient (as a function of composition and temperature) and the saturation pressure (as a function of temperature), and solve the above equation system for the four unknown molar fractions.

Furthermore, knowing the molar composition of ethanol on the liquid side, it is possible to calculate the mass fraction of ethanol $w_e$:

$$w_e = \frac{Mw_e}{Mw_e + \frac{(1 - x_e)}{x_e}Mw_w}$$

where $Mw_e$ and $Mw_w$ are, respectively, the molecular weights of ethanol and water.

It is thus possible to obtain the density of such a water-alcohol mixture at the reference temperature of 20 ° C using, for example, the tables provided by the "International Organization of Legal Metrology" in the "International Alcohol-ometric Tables". From the ratio between the mass fraction and the density it is possible to obtain the fraction (or percentage) by volume of ethanol in solution. It is important to refer to the density at 20 °C of the mixture and not to that measured as the% vol. is affected by the temperature variation and the result of the method and the instrument must be in line with the legislation concerning the indication of the volume degree of ethanol, which must be calculated at a temperature of 20 °C.

The vapor side concentration of ethanol can be calculated in mg/L using the ideal gas law:

$$C_{ethanol}^{VAP}\left[\frac{mg}{L}\right] = \frac{P\, y_e}{R_{gas}\, T} \times Mw_e \times 1000$$

The procedure using Dubowski's semi-empirical equation for the correlation of the concentration of the liquid side and the vapor side of ethanol, as well as the more complex calculation using the non-ideal liquid-vapor equilibrium (an example shown above) can be used both for the calculation of the concentration on the vapor side, the concentration on the liquid side is known and vice versa.

Further characteristics of the method according to the invention are set out in the subsequent claims.

The method of measuring the alcohol content of aqueous solutions by means of gas sensors according to the invention may be carried out in an apparatus such as the following one. In this apparatus, a closed tank containing the alcohol solution and headspace for the formation of the vapor phase, such as a 50 mL vessel filled with 30 mL of solution, is connected to the sampling device, analysis and quantification of ethanol by means of a pipeline, such as a 4 mm internal diameter silicone tube, connected at the top of the tank. The tank can have a vent into the atmosphere so as to maintain atmospheric pressure and a system for measuring the temperature of the solution.

The main line with the alcohol vapors is connected to a device capable of sucking this gaseous stream and mixing it (diluting it) with an air stream, such as a Venturi injector.

The air stream can be drawn by a pump or a compressor, such as a DC 6V diaphragm vacuum pump, and sent to the mixing and dilution device. To accelerate the establishment of the thermodynamic equilibrium between the liquid and vapor phases of the air, it can be bubbled into the liquid solution. The air/vapor dilution ratio can be modulated by acting on the mixing and dilution device and/or on the air flow rate and must be such as to generate a stream with an ethanol concentration measurable by the gas sensor used. The pumping device can be controlled through the use of a calculation module equipped with analog and/or digital pins for outgoing and incoming communications, such as a single board microcontroller and appropriate circuitry.

This stream of air enriched in alcohol can thus be sent to the analysis device in which the gas sensor is housed, or to a battery of sensors to increase the accuracy and instrumental precision, such as semiconductor sensors (type MQ- 3 or TGS-822), for the determination of ethanol. The measurement with these sensors can be designed in such a way as to alternate measurement phases with regeneration phases, thus having to alternate phases of introduction of air enriched in alcohol with phases of introduction of air only. These phases can be regulated by opening and closing the vapor suction line or by using another air pumping line. The control of devices such as pumps, compressors and valves can take place through the use of a calculation module equipped with analog and/or digital pins for output and input communications, such as a single board microcontroller and appropriate circuitry.

The gas sensors, hit by the gaseous stream, produce a response proportional to the concentration of ethanol present which can be processed by means of a calculation device, such as a single board microcontroller and suitable circuitry. The gas stream analyzed escapes from the analysis device and is released into the atmosphere.

As already noted, the concentration of ethanol measured in the gaseous phase can be obtained by analyzing hydroalcoholic solutions at known concentrations (in the vapor phase), and by constructing a calibration curve. The concentration in the vapor phase, starting from solutions with a known concentration in the liquid phase, can be obtained by means of thermodynamic models of the non-ideal liquid-vapor equilibrium type or with semi-empirical equations, such as the Dubowski's formula.

The measurement can be shown to the operator using a special device, such as an LCD screen, connected to the device in charge of the calculation and/or saved on a special storage device.

In more detail, the process for determining the ethyl alcohol of the invention can be described as comprising the following unitary operations:

- preparation and storage of hydroalcoholic solutions with a known concentration of ethanol;
- housing of the hydroalcoholic solution in a closed tank or with vent into the atmosphere, preferably with a volume of 50 mL and filled with 30 mL of solution, in order to allow the formation of the vapor phase; a pipe can be connected to the bottom of the tank for the introduction of air to be bubbled in the liquid phase in order to accelerate the establishment of the liquid-vapor equilibrium, the head of the tank being connected to the analysis device through a pipe, such as example a silicone tube;
- suction of the vapor phase and mixing with a stream of air through a mixing and dilution device, such as a venturi injector;
- pumping of the air stream rich in alcohol to the measuring device, which houses one or more gas sensors;
- measurement of the alcohol concentration in the gaseous phase and the temperature in the tank;
- pumping pure air into the measuring device in order to regenerate the gas sensors;
- construction of the calibration curve in order to relate the sensor signal with the alcohol concentration in the gaseous phase;
- use of thermodynamic models (non-ideal liquid-vapor equilibrium) or semi-empirical equations (eg Dubowski formula) to link the concentrations of ethanol in the vapor phase with those in the liquid phase;
- analysis of alcoholic solutions with unknown alcohol concentration.

## EXAMPLES

Some specific embodiments of the method for measuring ethyl alcohol in aqueous solutions through the use of sensors for gases according to the invention are described hereinafter merely by way of non-limiting examples, together with the results of the experiments. carried out.

## Process Flow Diagram

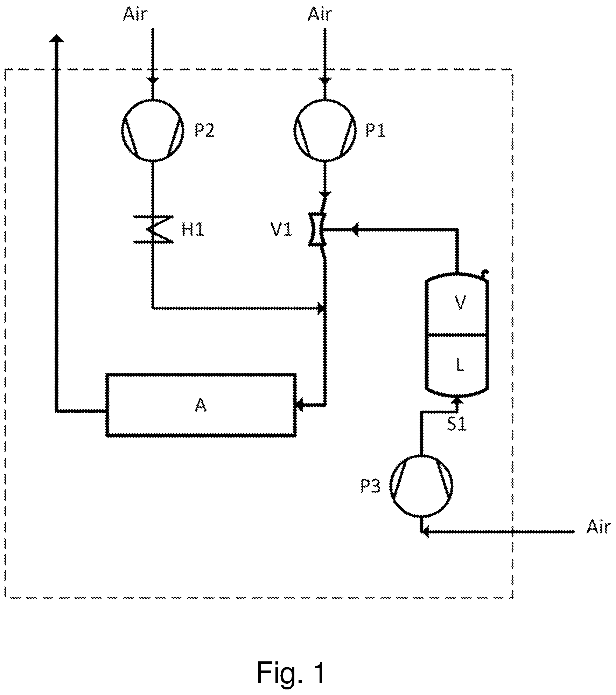

A process scheme according to the present invention is shown in **Figure 1.**

To implement the method of the invention, a hydroalcoholic solution is placed in the tank S1.

The thermodynamic equilibrium between the liquid phase and the vapor phase in the tank S1 is accelerated by blowing in air, by means of the pump P3. The vapor phase (V) of the alcoholic solution contained in the tank S1 is sucked by the Venturi injector V1 activated by the flow of air sucked in and pumped into the circuit by the membrane vacuum pump P1. The solution can be sent to analysis block A or further diluted through the air pumped by the vacuum pump P2. The regeneration phase of the gas sensors can be carried out by turning off pump P1 and turning on pump P2 so as to send pure air to the analysis block, possibly heated by the element H1.

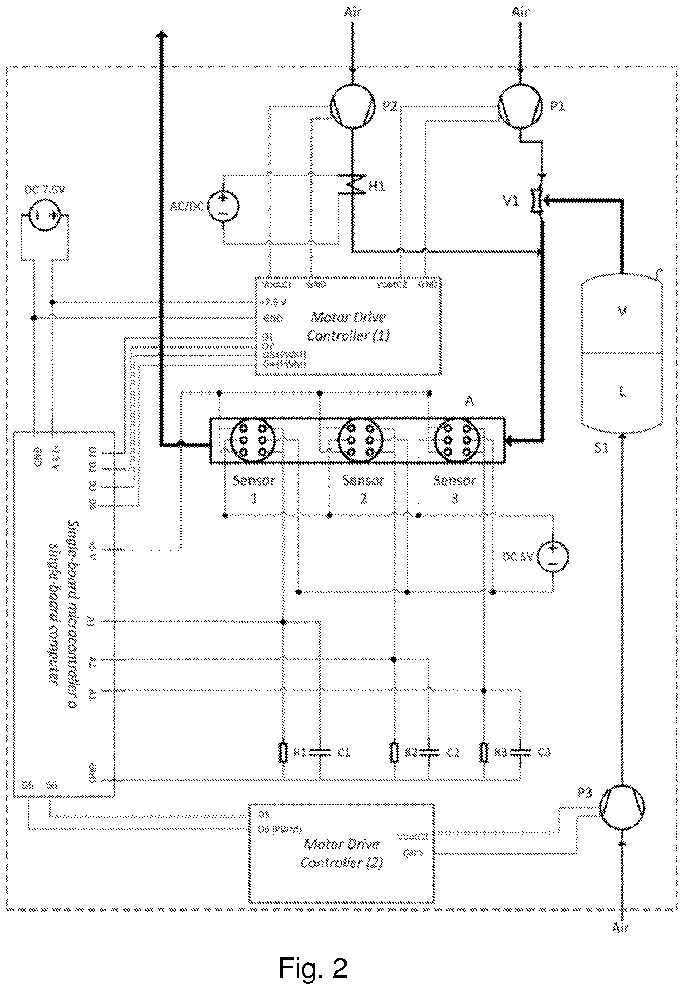

A piping and instrumentation diagram (also known as P&ID) is shown in **Figure 2.** The device in charge of the calculation, a single board microcontroller, powered with direct current at 7.5 V is pre-placed to control the device instrumentation. Through the use of a Motor Drive Controller, such as an L298N also powered by direct current and with 7.5 V, and through the use of PWM (Pulse-width modulation) digital signals, it is possible to control the " switching on and off of the vacuum pumps P1 and P2 and their power supply voltage, therefore their flow rate.

The gaseous stream generated in V1, resulting from the mixing and dilution of the stream coming from S1 and P1, is sent to the analysis block consisting of a battery of 3 gas sensors of the TGS-822 type (Sensor 1, Sensor 2 and Sensor 3). The heating resistor of the latter is powered by direct current at 5 V, with current control. The sensitive elements powered at 5 V by the single board microcontroller are connected respectively to resistors R1, R2, R3 to form voltage dividers, so as to quantify the change in resistance of the sensitive element due to the reaction with ethyl alcohol in terms of potential difference, using the analog input pins A1, A2, A3. Capacitors C1, C2, C3 serve as voltage regulators.

**Examples of determining the alcohol content**

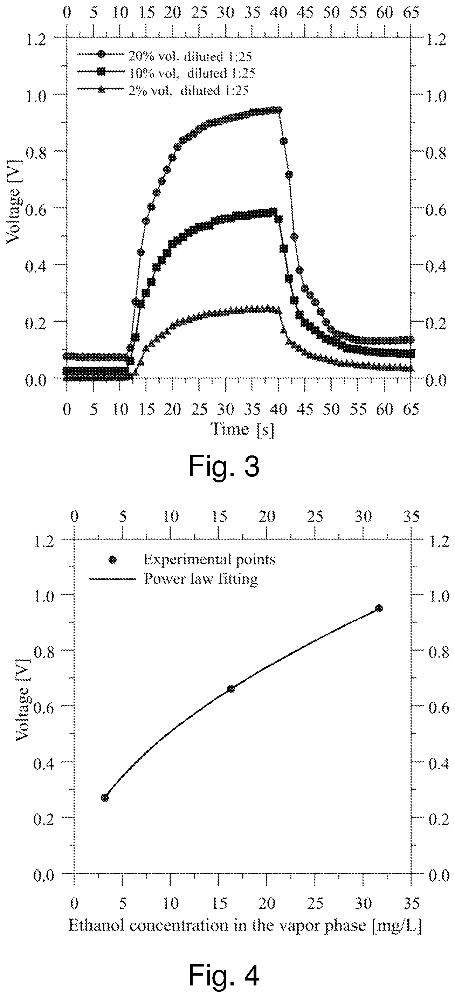

The measurement cycle includes a cleaning phase of 10 seconds in which pure air is sent to the analysis device (A in Figure 1 and Figure 2) using the vacuum pump P2 in Figure 1 and Figure 2. Al at the end of the cleaning phase, the actual measurement phase begins in which the stream of air enriched in ethanol coming from the venturi injector (V1 in Figure 1 and Figure 2) is sent to the analyzer for a time of 30 seconds. This is followed by the regeneration phase of the sensors with pumping of pure air by the vacuum pump P2 which lasts in this configuration 25 seconds, as shown in the diagram in **Figure 3.**

The following steps can be used to calibrate the instrument:

1. analysis of solutions of known titre, such as for example hydroalcoholic solutions at 2%, 10% and 20% vol., diluted 1 in 25 (Figure 3), measuring their voltages and temperatures;

2. iterative calculation of the mass concentration of ethanol in the liquid phase starting from the knowledge of the volume concentration in the liquid phase at the reference temperature of 20 ° C. The mass fraction $w_e$ is obtained from the equation $W_e = \dfrac{\%vol_e}{100} \dfrac{\rho_e}{\rho_{mix}}$ where $\rho_e$ is the density of pure ethanol and $\rho_{mix}$ is the density of the mixture, a function of the temperature and of the mass fraction itself;

3. once the mass fraction of ethanol is known, the mass concentration [g/L] of ethanol is calculated:

$$C_{ethanol}^{Liq} = w_e \, \rho_{mix} ;$$

4. being known the temperature of the analyzed solution, recorded by means of a suitable sensor, it is possible to calculate the concentration in the vapor phase [mg/L], for example using the Dubowski's equation:

$$C_{ethanol}^{Vap} = C_{ethanol}^{Liq} K_0 e^{A\,T}$$ where $K_0$ = 4.145 × 10$^{-2}$ [mg/g], $A$ = 0.06583 [1/°C] and T is the temperature of the analyzed solution [°C];

5. correlation, for example by means of a power law equation, of the concentration in the vapor phase with the voltage recorded by the measuring system **(Figure 4)**

Once the correlation between the vapor phase concentration and the sensor response (for example in terms of voltage) is known, it is possible to quantify the alcoholic strength of other hydroalcoholic solutions/commercial beverages. The procedure in this case can be as follows:

1. analysis of the solution having an unknown alcohol titer according to the same cycle used for calibration with recording of temperature and sensor response (e.g. voltage);

2. use of the correlation between vapor phase concentration and sensor response to calculate the vapor phase concentration $C_{ethanol}^{Vap}$ ;

3. calculation of the mass concentration in the solution using, for example, the Dubowski's inverse equation:

$$C_{ethanol}^{Liq} = C_{ethanol}^{Vap}/(K_0 e^{AT});$$

4. calculation of the mass fraction of ethanol and the density of the mixture at the reference temperature of 20 °C, as a function of the mass fraction itself, by iterative calculation;

5. Calculation of the alcoholic strength by volume.

**Comparison tests**

The following table shows some results obtained using the method thus described for the analysis of some commercial beverages of a known strength, with a medium-high alcohol content and deliberately selected in view if their complex composition.

| Sample | Declared alcohol content [%vol] | Allowed tolerance [%vol] (UE REGULATION No. 1169/2011, Annex XII) | Measured alchol content [%vol] |
|---|---|---|---|
| Aperol | 11.0 % | ± 1.0 % | 11.4 % |
| Baileys Irish Cream Original | 17.0 % | ± 1.0 % | 16.5 % |
| Birra Peroni | 4.7 % | ± 0.5 % | 4.8 % |
| Sparkling wine | 9.0% | ± 1.0 % | 9.4 % |
| Best Bräu Beer | 5.0% | ± 0.5 % | 5.2 % |

As in may be appreciated, the results are all very close to the alcoholic strength declared by the manufacturer, certainly within the tolerance range identified by EU Regulation No. 1169/2011 (Annex XII). This is also true for particularly complex alcoholic beverages, characterized by high concentrations of volatile organic compounds (e.g. Aperol), creamy milk-based solutions (e.g. Baileys: liqueur based on Irish whiskey and milk cream), beverages sparkling wines (e.g. sparkling wines and beers).

The above results demonstrate that the method and the device proposed by the present invention are able to overcome the criticalities that have characterized the other methods and devices of the prior art, thus being able to have a high industrial interest.

The present invention has been described with reference to some of its specific embodiments, but it is to be understood that variations or modifications may be made to it by those skilled in the art without thereby departing from its scope as defined by the attached claims.

**Claims**

1.   A method for determining the alcohol content of hydroalcoholic solutions or alcoholic beverages, comprising the following operations:

A) Calibration:

a) preparation of hydroalcoholic solutions with a known concentration of ethanol, said concentration of ethanol being in the range from 0 to 100% vol. of ethanol, and having a known temperature;
b) calculation of the concentration in the vapor phase of ethanol using liquid-vapor equilibrium curves for non-ideal mixtures, or using semi-empirical equations, such as Dubowski's formula;
c) aspiration and dilution in a gaseous phase of the vapor phase above the aforementioned hydroalcoholic solutions;
d) analysis of the gas phase concentration of ethanol using gas sensors;
e) construction of the calibration curve which links the sensor response to the concentration in the gas phase;

B) Analysis of samples of unknown alcohol content:

f) dilution of the sample in water to reduce the partial pressure of volatile organic compounds in the vapor phase, and measurement of its temperature;

g) aspiration and dilution in a gaseous phase of the vapor phase in the headspace of the hydroalcoholic solution resulting from the operation f);

h) analysis of the ethanol concentration in the gaseous phase resulting from operation g), using gas sensors and on the basis of the calibration curve of the operation e);

i) calculation of the ethanol concentration in the liquid phase using the same liquid-vapor equilibrium curves for non-ideal mixtures, or semi-empirical equations used for operation b).

2. A method for determining the alcohol content according to claim 1, wherein the concentration of ethanol in the starting sample to be analyzed is between 0.5 and 70% vol. of ethanol.

3. A method for determining the alcohol content according to claim 2, in which, in said concentration of ethanol it is comprised between 4.2 and 18% vol. of ethanol.

4. A method for determining the alcohol content according to any one of the preceding claims, wherein, in said sample analysis step f), said sample is diluted in water in a ratio between 1:10 and 1: 100.

5. A method for determining the alcohol content according to claim 4, wherein said dilution ratio in water is comprised between 1:25 and 1:50.

6. A method for determining the alcohol content according to any one of claims 1-5, wherein said gaseous phase of operations c) and g) is air, and the aspiration and dilution of the vapor phase above said solutions occurs through one or more pumps or compressors with an air/vapor dilution ratio that can be modulated and adjusted by acting on a mixing device and/or on the air flow rate.

7. A method for determining the alcohol content according to any one the preceding claims, wherein the analysis of the sensor response comprises a phase of processing of said response, preferably a computer processing or fitting thereof.


**Patentansprüche**

1. Ein Verfahren zur Bestimmung des Alkoholgehalts von hydroalkoholischen Lösungen oder alkoholischen Getränken, umfassend die folgenden Schritte:

A) Kalibrierung:

a) Herstellung von hydroalkoholischen Lösungen mit einer bekannten Ethanolkonzentration, wobei die genannte Ethanolkonzentration im Bereich von 0 bis 100 Vol.-% Ethanol liegt und eine bekannte Temperatur aufweist;

b) Berechnung der Ethanolkonzentration in der Dampfphase unter Verwendung von Flüssigkeits-Dampf-Gleichgewichtskurven für nicht-ideale Gemische oder unter Verwendung von semi-empirischen Gleichungen, wie der Dubowski-Formel;

c) Ansaugen und Verdünnen der Dampfphase in einer Gasphase oberhalb der vorgenannten hydroalkoholischen Lösungen;

d) Analyse der Gasphasenkonzentration von Ethanol unter Verwendung von Gassensoren;

e) Erstellung der Kalibrierkurve, welche die Sensorantwort mit der Konzentration in der Gasphase verknüpft;

B) Analyse von Proben mit unbekanntem Alkoholgehalt:

f) Verdünnung der Probe in Wasser zur Reduzierung des Partialdrucks von flüchtigen organischen Verbindungen in der Dampfphase und Messung ihrer Temperatur;

g) Ansaugen und Verdünnen der Dampfphase in einer Gasphase im Kopfraum der aus dem Schritt f) resultierenden hydroalkoholischen Lösung;

h) Analyse der Ethanolkonzentration in der Gasphase, resultierend aus dem Schritt g), unter Verwendung von Gassensoren und auf der Grundlage der Kalibrierkurve des Schrittes e);

i) Berechnung der Ethanolkonzentration in der Flüssigphase unter Verwendung der gleichen Flüssigkeits-Dampf-Gleichgewichtskurven für nicht-ideale Gemische oder der für den Schritt b) verwendeten semi-empirischen Gleichungen.

**2.** Ein Verfahren zur Bestimmung des Alkoholgehalts nach Anspruch 1, wobei die Ethanolkonzentration in der zu analysierenden Ausgangsprobe zwischen 0,5 und 70 Vol.-% Ethanol liegt.

**3.** Ein Verfahren zur Bestimmung des Alkoholgehalts nach Anspruch 2, bei dem die genannte Ethanolkonzentration zwischen 4,2 und 18 Vol.-% Ethanol liegt.

**4.** Ein Verfahren zur Bestimmung des Alkoholgehalts nach einem von 5 der vorhergehenden Ansprüche, wobei in dem genannten Probenanalyseschnitt f) die genannte Probe in Wasser in einem Verhältnis zwischen 1:10 und 1:100 verdünnt wird.

**5.** Ein Verfahren zur Bestimmung des Alkoholgehalts nach Anspruch 4, wobei das genannte Verdünnungsverhältnis in Wasser zwischen 1:25 und 1:50 liegt.

**6.** Ein Verfahren zur Bestimmung des Alkoholgehalts nach einem von 10 Ansprüchen 1 bis 5, wobei die genannte Gasphase der Schritte c) und g) Luft ist und das Ansaugen und Verdünnen der Dampfphase oberhalb der genannten Lösungen durch eine oder mehrere Pumpen oder Kompressoren mit einem Luft/Dampf-Verdünnungsverhältnis erfolgt, das durch Einwirken auf eine Mischeinrichtung und/oder auf die Luftdurchflussrate moduliert und eingestellt werden kann.

**7.** Ein Verfahren zur Bestimmung des Alkoholgehalts nach einem von 15 der vorhergehenden Ansprüche, wobei die Analyse der Sensorantwort eine Phase der Verarbeitung der genannten Antwort, vorzugsweise eine Computerverarbeitung oder Anpassung (Fitting) davon, umfasst.

## Revendications

**1.** Procédé de détermination de la teneur en alcool de solutions hydroalcooliques ou de boissons alcoolisées, comprenant les étapes suivantes :

A) Étalonnage :

a) préparation de solutions hydroalcooliques présentant une concentration connue en éthanol, ladite concentration étant comprise entre 0 et 100 % en volume d'éthanol, et présentant une température connue ;
b) calcul de la concentration d'éthanol dans la phase vapeur à l'aide de courbes d'équilibre liquide-vapeur pour des mélanges non idéaux, ou à l'aide d'équations semi-empiriques, telles que la formule de Dubowski ;
c) aspiration et dilution, dans une phase gazeuse, de la phase vapeur située au-dessus desdites solutions hydroalcooliques ;
d) analyse de la concentration d'éthanol dans la phase gazeuse à l'aide de capteurs de gaz ;
e) établissement d'une courbe d'étalonnage reliant la réponse du capteur à la concentration dans la phase gazeuse ;

B) Analyse d'échantillons à teneur en alcool inconnue :

f) dilution de l'échantillon dans de l'eau, de manière à réduire la pression partielle des composés organiques volatils dans la phase vapeur, et mesure de sa température ;
g) aspiration et dilution, dans une phase gazeuse, de la phase vapeur présente dans l'espace de tête de la solution hydroalcoolique obtenue à l'étape f) ;
h) analyse de la concentration d'éthanol dans la phase gazeuse obtenue à l'étape g), au moyen de capteurs de gaz et sur la base de la courbe d'étalonnage de l'étape e) ;
i) calcul de la concentration d'éthanol dans la phase liquide à l'aide des mêmes courbes d'équilibre liquide-vapeur pour des mélanges non idéaux, ou des équations semi-empiriques utilisées à l'étape b).

**2.** Procédé de détermination de la teneur en alcool selon la revendication 1, dans lequel la concentration en éthanol de l'échantillon initial à analyser est comprise entre 0,5 et 70 % en volume d'éthanol.

**3.** Procédé de détermination de la teneur en alcool selon la revendication 2, dans lequel ladite concentration d'éthanol est comprise entre 4,2 et 18 % en volume d'éthanol.

4. Procédé de détermination de la teneur en alcool selon l'une quelconque des revendications précédentes, dans lequel, lors de l'étape f) d'analyse de l'échantillon, ledit échantillon est dilué dans de l'eau selon un rapport compris entre 1:10 et 1:100.

5. Procédé de détermination de la teneur en alcool selon la revendication 4, dans lequel ledit rapport de dilution dans l'eau est compris entre 1:25 et 1:50.

6. Procédé de détermination de la teneur en alcool selon l'une quelconque des revendications 1 à 5, dans lequel la phase gazeuse des étapes c) et g) est constituée d'air, et dans lequel l'aspiration et la dilution de la phase vapeur située au-dessus desdites solutions sont effectuées au moyen d'une ou de plusieurs pompes ou compresseurs, le rapport de dilution air/vapeur pouvant être modulé et ajusté par action sur un dispositif de mélange et/ou sur le débit d'air.

7. Procédé de détermination de la teneur en alcool selon l'une quelconque des revendications précédentes, dans lequel l'analyse de la réponse du capteur comprend une étape de traitement de ladite réponse, de préférence un traitement ou un ajustement informatisé de celle-ci.

Fig. 1

Fig. 2

Fig. 3

Fig. 4

## REFERENCES CITED IN THE DESCRIPTION

### Patent documents cited in the description

- EP 0357027 B1
- US 20040157334 A, Barashkov
- US 5091155 A, Takayama
- WO 9013026 A
- IT 102017000138957, Caccavo

### Non-patent literature cited in the description

- **ZOECKLEIN, BW**. Production Wine Analysis. Springer-Verlag, New York Inc., 2012
- **WILLIAMS, MB** ; **REESE, HD**. Colorimetric determination of ethyl alcohol. *Anal. Chem.*, 1950, vol. 22 (12), 1556-1561
- **FALQUÉ LÓPEZ, E.** ; **FERNÁNDEZ GÓMEZ, E.** Simultaneous determination of the major organic acids, sugars, glycerol, and ethanol by HPLC in grape musts and white wines. *J. Chromatogr. Sci.*, 1996, vol. 34 (5), 254-257
- **ZHANG, Y. et al.** A new method study of spectral measurement and prediction based on the non-linear solution concentration of alcohol. *Physica B: Condensed Matter*, 2017, vol. 516, 32-35
- **VORLOP, KD et al.** On-line measurement of ethanol with a gas-sensor-dip-electrode. *Biotechnology Letters*, 1983, vol. 5 (8), 509-514
- **VERNIER**. *Ethanol Sensor Eth-Bta.*, 15 September 2017, https://www.vernier.com/products/sensors/eth- bta
- **BROWNE, A. et al.** Development of a Sturdy, Portable Instrument for the Rapid Deter-mination of Alcohol in Beer. *ASBC Journal*, 1991, vol. 49 (2), 74-77
- **AB MUTALIB, NA et al.** IIUM-Fabricated Portable Electronic Nose for Halal Authentication in Beverages. *5th International Conference on Information and Communication Technology for the Muslim World (ICT4M)*, 2013, vol. 1-4